# EUROPEAN PATENT SPECIFICATION

(11) **EP 1 687 636 B1**
(45) Date of publication and mention of the grant of the patent: **14.01.2009**
(21) Application number: 04787562.0
(22) Date of filing: 29.09.2004
(51) Int. Cl.: G01N 33/68, C07K 14/475, G01N 33/543

(54) **RAPID DETERMINATION OF DIFFERENT FORMS OF HEPATOCYTE GROWTH FACTOR (HGF) IN THE BODY FLUIDS**
SCHNELLE BESTIMMUNG VERSCHIEDENER FORMEN VON HGF (HEPTOCYTE GROWTH FACTOR) IN DEN KÖRPERFLÜSSIGKEITEN
DETERMINATION RAPIDE DE DIFFERENTE FORMES DU FACTEUR DE CROISSANCE DES HEPATOCYTES (HGF) DANS LES LIQUIDES ORGANIQUES

(30) Priority: 29.09.2003 US 507192 P
(43) Date of publication of application: 09.08.2006
(73) Proprietor: PEAS Institut AB, 587 23 Linköping (SE)
(72) Inventor: Nayeri, Fariba, 582 16 Linköping (SE); Nayeri, Tayeb, 582 47 Linköping (SE)
(74) Representative: Lindgren, Anders
(86) International application number: PCT/IB2004/003606
(87) International publication number: WO 2005/031365

(56) References cited:
- WO-A-01/44294
- WO-A-02/17964
- NAYERI FARIBA ET AL: "Sample handling and stability of hepatocyte growth factor in blood samples" CYTOKINE, vol. 19, no. 4, 21 August 2002 (2002-08-21), pages 201-205, XP002322213 ISSN: 1043-4666
- MATSUNO MITSUTAKA ET AL: "Clinical evaluation of hepatocyte growth factor in patients with gastrointestinal and pancreatic diseases with special reference to inflammatory bowel disease" RESEARCH COMMUNICATIONS IN MOLECULAR PATHOLOGY AND PHARMACOLOGY, vol. 97, no. 1, 1997, pages 25-27, XP008044746 ISSN: 1078-0297
- SHIMADA YOSHIHISA ET AL: "Hepatocyte growth factor production may be related to the inflammatory response in patients with acute myocardial infarction" CIRCULATION JOURNAL, vol. 66, no. 3, March 2002 (2002-03), pages 253-256, XP002322219 ISSN: 1346-9843
- BORAWSKI JACEK ET AL: "Serum hepatocyte growth factor is associated with viral hepatitis, cardiovascular disease, erythropoietin treatment, and type of heparin in haemodialysis patients" NEPHROLOGY DIALYSIS TRANSPLANTATION, vol. 17, no. 4, April 2002 (2002-04), pages 637-644, XP002322220 ISSN: 0931-0509
- NAYERI F ET AL: "High serum hepatocyte growth factor levels in the acute stage of community-acquired infectious diseases" SCANDINAVIAN JOURNAL OF INFECTIOUS DISEASES, vol. 34, no. 2, 2002, pages 127-130, XP008044743 ISSN: 0036-5548
- SRIVASTAVA MAYA ET AL: "Elevated serum hepatocyte growth factor in children and young adults with inflammatory bowel disease" JPGN JOURNAL OF PEDIATRIC GASTROENTEROLOGY AND NUTRITION, vol. 33, no. 5, November 2001 (2001-11), pages 548-553, XP008044741 ISSN: 0277-2116
- HUANG MING-SHYAN ET AL: "Serum hepatocyte growth factor levels in patients with inflammatory lung diseases" KAOHSIUNG JOURNAL OF MEDICAL SCIENCES, vol. 15, no. 4, April 1999 (1999-04), pages 195-201, XP008044745 ISSN: 0257-5655
- KUNO YUJI ET AL: "Possible involvement of neutrophil elastase in impaired mucosal repair in patients with ulcerative colitis." JOURNAL OF GASTROENTEROLOGY, vol. 37, no. Supplement 14, 2002, pages 22-32, XP008044736 ISSN: 0944-1174

## Description

### FIELD OF THE INVENTION

The present invention is based on the discovery that the configuration, properties and activity of hepatocyte growth factor (HGF) differ between different inflammatory diseases. The invention is directed to diagnosis and/or monitoring of different inflammatory diseases. Therefore the present invention relates to a method for rapid detection (within 1-7 minutes), of amounts, as well as discrimination between different forms of, HGF that are over-presented in body fluids with different degrees of biological activity. The present invention further relates to diagnosing and monitoring of inflammatory disorders in various organs, for example, bowel and skin by evaluation of the amounts and over presenting forms of HGF in body fluids of an injured organ.

### BACKGROUND OF THE INVENTION

Hepatocyte growth factor (HGF) is a unique growth factor, which is unrelated to other well-known polypeptide mitogens. It is a protein expressed in the mesenchymal cells such as lung macrophages and fibroblasts (Yanagita K, et al. J Biol Chem. 1993, 268). Kupffer cells in the liver and leukocytes (Sakaguchi H. et al. Hepatology 19, 1994, Noji S, et al. Biochem Biophys Res Commun 173, 1993). HGF is secreted in response to cell damage and appears to be important for the regeneration of certain organs and healing of wounds (Arakaki N et al. Hepatology 221, 1995). It is a heterodimer, having disulphide bonded heavy and light chains of approximately 60 and 30 kDa respectively, first synthesized as an inactive precursor (Miyazawa K et al. J Biol Chem 268,[A1] 1994). The precursor is cleaved to an active protein in the damaged organ by a specific activator (Naka D et al. JBiol Chem 276, 1992, Nishizaki [A2]T et al. J Am Coll Surg 181, 1995). HGF acts paracrinally, i.e. it affects adjacent cells, as well as endocrinally, i.e. it has a long-distance effect (Yanagita K. et al. Biochem Biophys Res Commun 182, 1992, Kono S et al. Biochem Biophys Res Commun 186, 1992). The target cells of HGF are fully developed epithelial cells (Matsumoto K and Nakamura T. J Gastroenterology [A3]Hepatology 6, 1991). HGF is produced and is present in high concentrations at sites of organ damage (Nayeri F et al. Scand JInfect Dis, 34, 2002).

The systemic and local production of HGF in various infectious diseases has been studied and high serum HGF concentrations have been observed during acute infectious diseases such as gastroenteritis, sepsis, pneumonia, skin and soft tissue infections and pyelonephritis (Nayeri F et al. Scand J Infect Dis, 34, 2002). Simultaneous with enhanced systemic production of HGF, high HGF concentrations have been found in cerebrospinal fluid during meningitis (Nayeri F et al. J Infect Dis, 181, 2000). Raised HGF concentrations in exhaled breath condensate (Nayeri F, et al. Respir Med, 96, 2002) in patients with pneumonia, which had no correlation to serum levels of HGF, indicated a local production of HGF during pneumonia. Furthermore the stability of HGF in serum has been studied (Nayeri F, et al, CYTOLIKE, 2002) and HGF was found to be very stable in diluted feces samples and several freeze-thaw cycles, different buffers or several years of storage at -20 °C did not affect feces HGF concentrations significantly. High amounts of HGF in feces during diarrhea have been shown to possibly indicate that patient suffers from a transmittable gastroenteritis (PCT application Ser. No.p16114PC-00 and WO 02/17964). Further, monitoring of HGF levels before and after treatment during infectious diseases have been shown to possibly reveal therapeutic failure at an early stage (PCT application Ser. No.p16114PC-00).

### SUMMARY OF THE INVENTION

In one embodiment, the invention is directed to a method for diagnosing an inflammatory disorder in an individual, and comprises contacting a body fluid sample from the individual with an immunoassay device to determine relative levels of different forms of hepatocyte growth factor (HGF) in the sample, and correlating the determined levels to an inflammatory disorder. The present invention provides a method for rapid determination and discrimination between different forms of HGF in serum/plasma as well as in the other body fluids, such as, for example, stool, urine, cerebrospinal fluid, exhaled breath condensate, semen, saliva, joint fluid and ulcer secretion. Knowledge of the concentration and quality of HGF facilitates diagnosis and monitoring of diseases in which presence of HGF may play an important role.

Running the sample from channels that are immobilized respectively by anti-HGF monoclonal antibodies, met proto-oncogene receptor (c-met), and carboxymethyl dextran gives information about amount and quality of HGF in the sample at the same time. To overcome the non-specific reactions in the sample and reveal the back ground signals, dextran may optionally be added to sample that binds to HGF and diminishes the signals. Substraction of results from the samples before and after adding dextran give more reliable information about the amounts of HGF.

The present invention also relates to a method of diagnosing inflammatory bowel diseases by evaluation of different forms of HGF amounts and quality (over presenting form of HGF) in feces samples.

In a further embodiment, the present invention relates to evaluation of ulcers by examination of the amount and quality of different forms of HGF in the ulcer secrete.

In another embodiment, the present invention relates to a method of diagnosis of different inflammatory disorders in CNS by examination of different forms of HGF amounts and quality in the cerebrospinal fluid.

In another embodiment, the present invention relates to diagnosis and evaluation of therapy in inflammatory diseases and traumatic injuries in the lungs by examination of different forms of HGF amounts and quality (over presentating form of HGF) in bronchoalveolar lavage, sputum or exhale breath condensate.

In another embodiment, the present invention relates to diagnosis of inflammatory disorders in kidneys by evaluation of different forms of HGF amounts and quality in urine.

In yet another embodiment the present invention relates to a method for diagnosis of periodontal disorders by examination of different forms of HGF amounts and quality in saliva.

In another embodiment, the present invention relates to diagnosis of inflammatory disorders in peritoneum, pericardium and pleura by analysis of peritoneal fluid, pericardial exudate or pleural exudate regarding to different forms of HGF amounts and quality.

In another embodiment, the present invention relates to a method for differential diagnosis between disorders in joints caused by infection or other inflammatory disorders by evaluation of different forms of HGF amounts and over presenting forms in the joint fluid.

In another embodiment, the invention is directed to an immunoassay device for diagnosing an inflammatory disorder in an individual, comprising at least two test areas having immobilized respectively therein reagent for binding different forms of hepatocyte growth factor (HGF). In a more specific embodiment, the device is a multichannel device.

Various other objects and advantages of the present invention will become apparent from the drawings and the following description of the invention.

### BRIEF DESCRIPTION OF THE DRAWINGS

The following detailed description will be more fully understood in view of the drawings, in which:
Fig. 1 shows the individual levels of feces HGF in different groups;
Fig. 2 shows a demonstration of feces of a patient with *Salmonella Enteritis with Western blotting;*
Fig. 3 shows relevant differences in feces HGF levels compared to baseline;
Fig. 4 shows relevant differences in feces levels after several freeze-thaw cycles compared to the baselines;
Fig. 5 shows the expression of c-met HGF receptor in human skin biopsies;
Fig. 6 shows a demonstration of HGF in ulcer secretes of a patient with chronic leg ulcer with Western blotting;
Fig. 7 shows an expression of HGF and an expression of met-proto-oncogene receptor in a patient;
Fig. 8 is an SPR sensogram showing the signal obtained from analysis of feces of patients with infectious gastroenteritis;
Fig. 9 is a histogram showing the differences between cycle responses determined by SPR in different groups of infectious gastroenteritis, normal healthy controls, and patients with colon cancer;
Fig. 10 demonstrates a correlation between the concentration of HGF in feces during infectious gastroenteritis obtained by ELISA and cycle responses by SPR;
Fig. 11 is a histogram showing the effects of increase of the immobilization levels by increasing the contact time during immobilization;
Fig. 12 shows SPS-PAGE of feces;
Fig. 13 shows an SPR sensogram from feces analysis;
Fig. 14 is a histogram showing the differences between the SPR signal from patients with different disorders; and
Fig. 15 shows a typical sensogram of human ulcer secrete in nine patients with chronic leg ulcers.

### DETAILED DESCRIPTION OF THE INVENTION

Recognizing the clinical importance and differences between recommended therapies, differential diagnosis between inflammatory disorders in the body has been the subject of several investigations. One major clinical problem is determining whether infection or other inflammatory disorders cause the disease. There are several markers that typically are used by physician to establish the right diagnosis such as microscopic analysis and culture of body fluids, white blood cell count, C-reactive protein, plasma procalcitonin and lactate. However, there are still no golden standards to be used. Problems in establishing correct diagnosis occurs daily while treating inflammatory disorders in bowel, ulcers, joint diseases, CNS disorders, peritoneal, pleural and pericardial effusions, among others. The amounts of routine markers such as CRP and WBC might be high in several disorders and cultures are not always positive in spite of an infection. High amounts of HGF and its application in diagnosis and prognosis of infectious diseases are discussed in PCT application Ser. No.p16114PC-00. Yet in these studies, the whole amount of HGF in the body fluids was determined by ELISA method.

In the present invention methods quantitatively determination of HGF's conducted and additionally different forms of HGF found in the body fluids at the same time are distinguished. One can evaluate the quality of HGF in the body fluids and differentiate between infection and other inflammatory disorders in the organs at an early stage.

Various studies about HGF have been reported. Some studies have used determination of HGF in plasma/serum and urine for diagnosis and screening of diseases such as acute renal deficiency (Taman M et al. Clin Nephrol, 48, 1997)*,* myocardial infarction (Matsumori A et al. Biochem Biophys Res Commun, 221, 1996), carcinoma of bladder (Rosen EM et al J Urol, 157, 1997), acute pancreatitis (Ueda T et al. J Gastroenterol. 32, 1997) and acute and chronic lung diseases (Yamanouchi H et al. Respir Med, 92, 1998*,* Maeda J et al. Am J Respir Crit Care Med, 152, 1995*).* For this reason, previously described methods such as ELISA and Western blotting have been used.

HGF has been investigated during infectious diseases. HGF levels have been found to increase in blood during pneumonia, meningitis, skin and soft tissue infection, gastroenteritis, sepsis and pyelonephritis (Nayeri F et al. Scand J Infect Dis, 34, 2002). Further, levels of HGF have been found to increase locally at the site of infection such as in exhaled breath condensate during pneumonia (Nayeri F, et al. Respir Med, 96, 2002), in ulcer secrete during ulcer infection, in feces during acute gastroenteritis, in urine during pyelonephritis and in cerebrospinal fluid during acute bacterial meningitis (Nayeri F et al. J Infect Dis, 181, 2000). Detection of high amounts of cytokines during inflammatory diseases is not a unique finding. However, in some cases, determination of HGF has been found to to be a sensitive method that could detect specific clinical problems much easier than the routine methods (PCT application Ser. No.p16114PC-00).

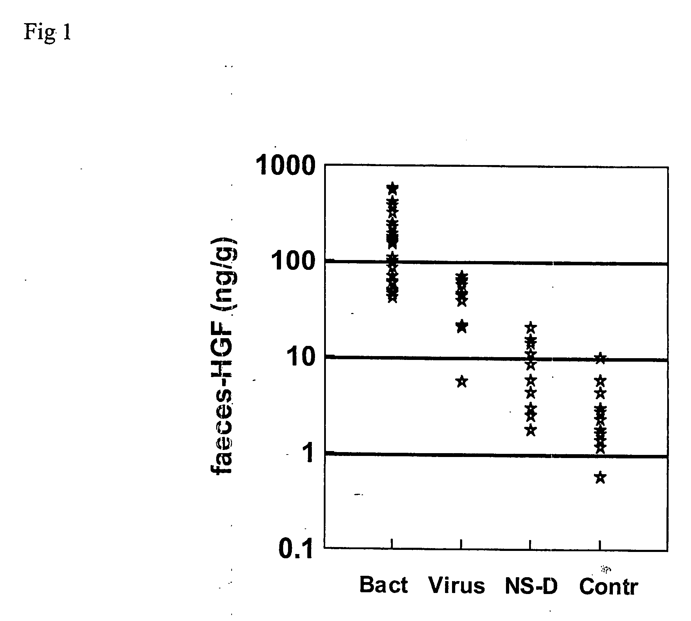

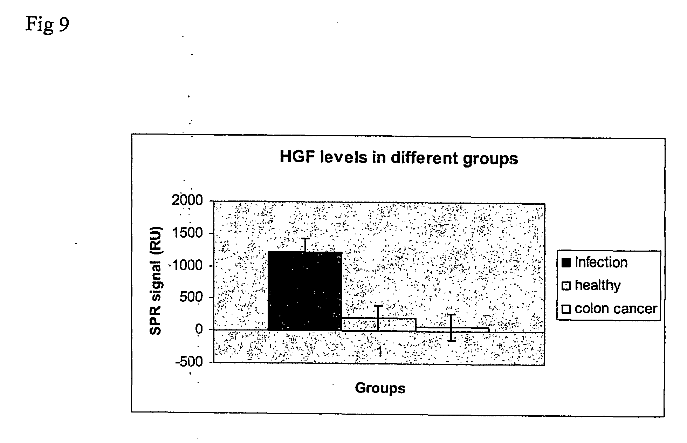

Patients who are admitted to the hospital because of an acute episode of diarrhea are isolated until they are recovered or until it is clear that they do not suffer from a transmittable gastroenteritis. It has happened that in some cases needing acute surgical intervention, the diagnosis and treatment have been postponed because the patients were admitted with atypical symptoms such as diarrhea. Levels of HGF in feces increase during acute infectious gastroenteritis but the HGF levels are low in unspecific diarrhea (Fig 1,9) (PCT application Ser. No.p16114PC-00).

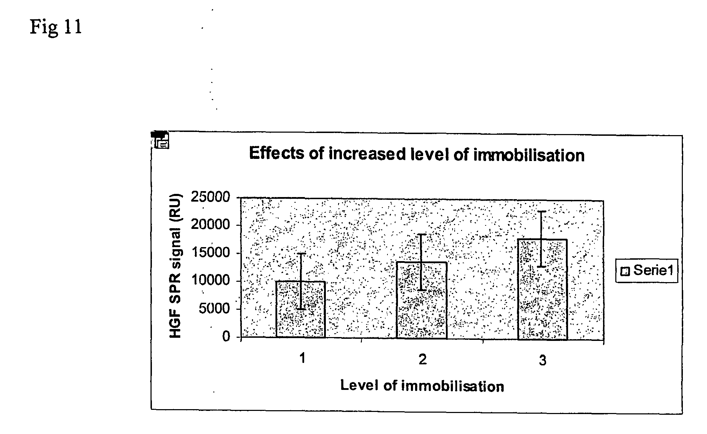

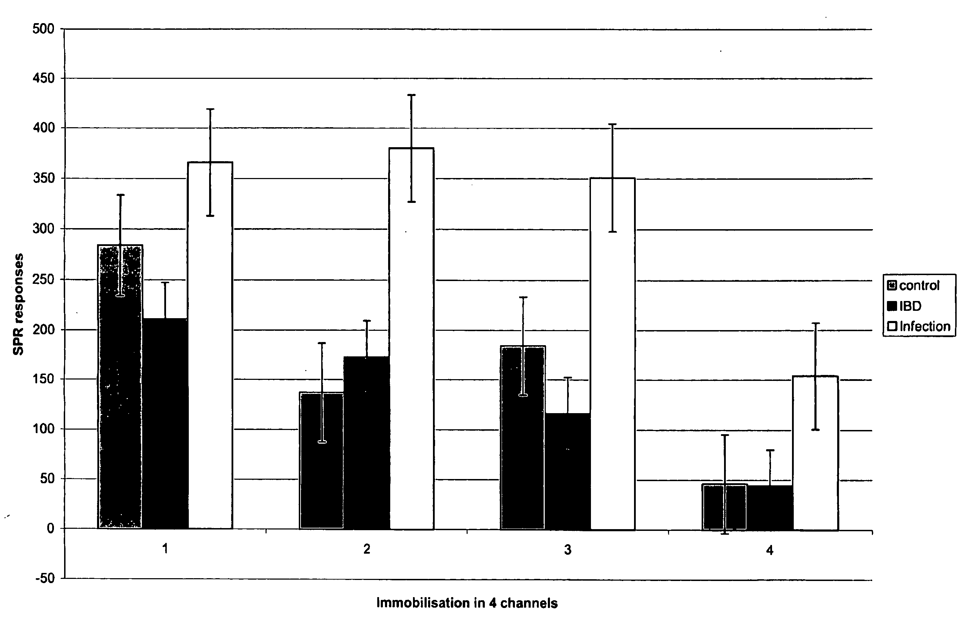

It has now been determined that the quality or form of HGF in the feces is another marker that contributes to diagnosis of the nature of bowel diseases. The single chain HGF precursor is found in high levels in feces during acute gastroenteritis. This form of HGF has high affinity to monoclonal anti HGF antibodies as well as to dextran (Fig 14). The affinity to monoclonal anti HGF antibodies is correlated to the immobilization level (Fig 11). On the other hand, a higher level of another form of HGF is observed in the feces during chronic inflammatory bowel diseases such as Crohn disease. This form of HGF has no or little affinity to dextran but binds to c-met receptor better than it binds to monoclonal anti HGF antibodies (Fig. 14). The affinity of this form of HGF to c-met receptor correlates to the immobilization level. Thus, determination of HGF in feces can be used as a diagnostic marker in bowel diseases.

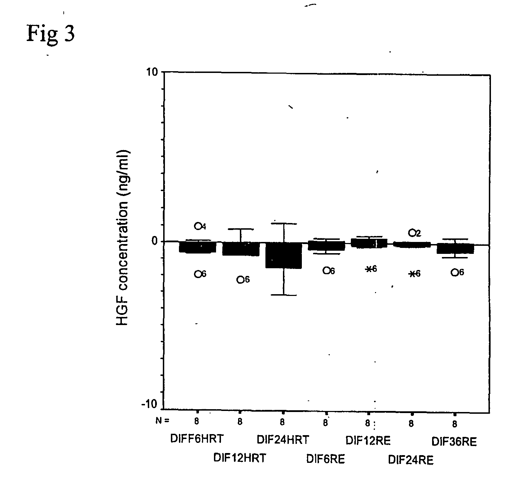

Further, unlike other cytokines HGF is very stable in the reconstructed (diluted 1:6 in buffer) feces. Various mechanical and chemical strains relevant to sample handling do not affect the fecal HGF concentration significantly. Storage at different temperatures and different periods of storage, several freeze-thaw cycles, freeze-drying and the buffer used do not influence the feces HGF results significantly (Fig 3-4). Processing the fecal samples with protease inhibitor does not influence the HGF concentration. The stable structure of HGF in spite of the constituent elements of feces might be the reason for such stability. This observation is important when planning comparable studies.

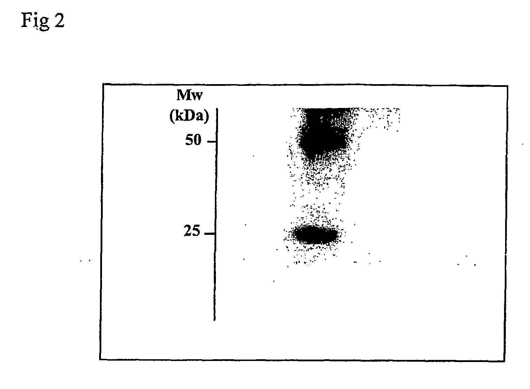

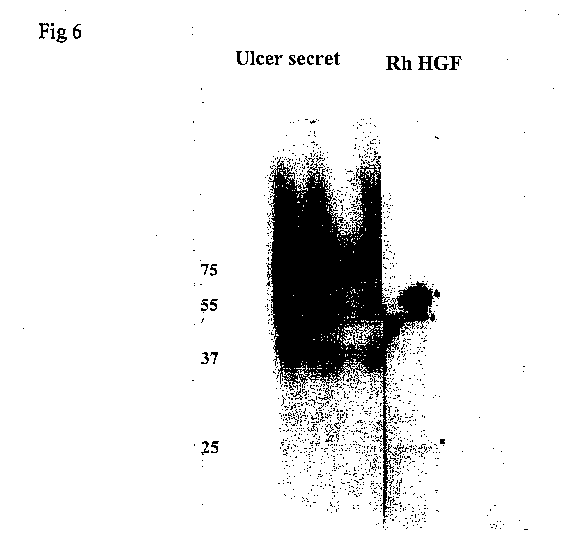

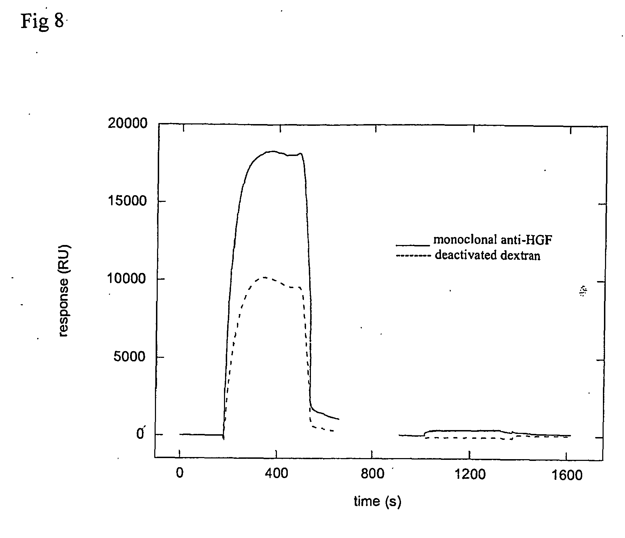

Thus, the present invention relates to a rapid method for detection of level and quality or form of different forms of HGF in feces. The previous described methods such as ELISA and Western blotting are based on an interaction between HGF in the samples and an antibody that binds specifically to HGF. In ELISA, the amount of HGF single-chain and double-chain is determined. By Western-blotting the quality of HGF in the body fluid is determined by detection of apparent molecular masses present in the sample (Fig 2,6). However the methods are cumbersome and laborious. In the present invention, in one embodiment, surface plasmon resonance (BIACORE) method can be used for the first time for detection of different forms of HGF in feces. The innovative use of biosensors is useful, inexpensive and rapid in this area of analysis. The technique is able to detect HGF levels and quality in a single run. The biosensor is defined as an analytical device consisting of a re-usable immobilized biological ligand, in this case monoclonal antibodies against HGF and c-met proto-oncogene HGF receptor that senses the analyte (HGF), and a physical transducer, which translates this phenomenon into an electric signal (Fig 8). The output values from the Biacore device may also be formatted into numbers, wherein each channel can be assigned a number based on concentration and affinity after a run. Comparing the values from different test results to each other can reveal the character of a sample. Additionally, the numbers can be formatted to a digital picture or color. For example, if "RGB" consists of a Red, Green, Blue system, or "CMYK" consists of a Cyan, Magenta, Yellow, Black, all of which are standard printing colors, the numbers from the channels from a test device can be correlated with either the RGB or the CMYK spectrum, and give a color distinguishable test result. The range of colors could be interpreted to simplified answers to evaluate the samples visually.

### Example:

Channel 1 : 250 RU CYAN
Channel 2 : 400 RU MAGENTA
Channel 3 : 300 RU YELLOW
Channel 4: 150 RU BLACK.

In one embodiment, feces samples are prepared by diluting an exact amount of feces in buffer such as distilled water or PBS containing protease inhibitor in a concentration between 1-5%. The protease inhibitor cocktail must be able to inhibit the different proteases in feces. The preparation is centrifuged 1000-10,000 G and the supernatant is used for detection of HGF by BIACORE. The process might take between 1-7 minutes for each sample. Recognizing that HGF binds to dextran, it might be difficult to evaluate results because HGF (found during infection) binds with a relative high affinity to the dextran layer, which is used as a control non-immobilized channel. To overcome this problem, carboxymethyl dextran 5% may be added to the samples. Running a part of one sample diluted 1:2 with distilled water and another part of the same sample combined with carboxymethyl dextran and subscribing the results from each other, the real levels of HGF can be detected in the samples.

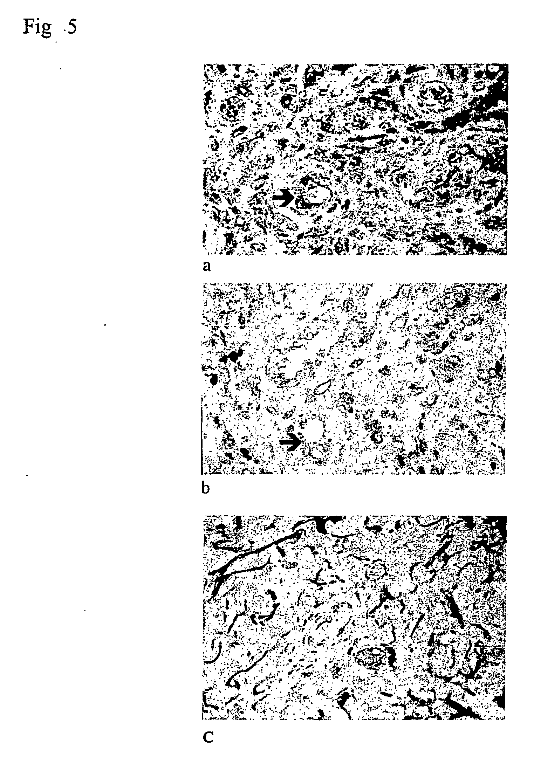

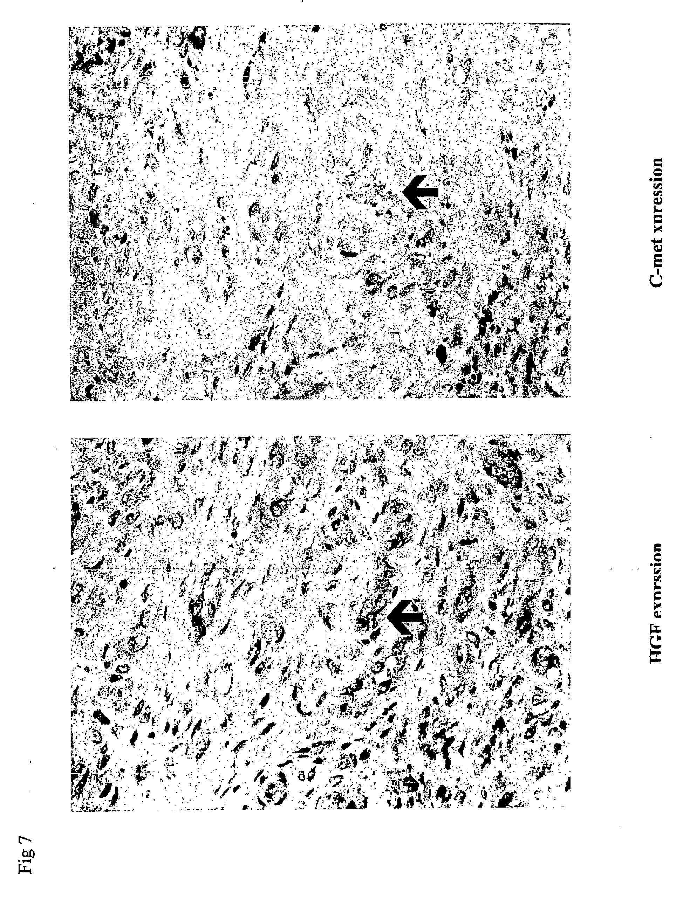

Treatment with exogenic HGF has been shown to be beneficial in treatment of some cases of chronic leg ulcers (Nayeri F et al. J Dermatol Treat, 13, 2002)(PCT application Ser. No.p16114PC-00). HGF has been found to enhancd migration of healthy neighbor skin epithelial cells towards the damaged area by changing the cytoskletal structure of cells in vitro. We have recently shown an enhanced expression of met proto-oncogene receptor (c-met) in the ulcer area of patients with chronic ulcers. Treatment with exogenous HGF decreased c-met expression significantly (Fig 5). There was a negative correlation between endogenous HGF concentration in the ulcer secrete and met proto-oncogene receptor (c-met) expression (Fig 7). Treatments with exogenous HGF in the patients with a low amount of endogenous HGF and high met proto-oncogene receptor (c-met) expression caused vascular proliferation and ulcer area reduction. Determination of HGF in ulcer secrete contributes to evaluation of ulcer and recognition of patients in whom HGF treatment might be beneficial.

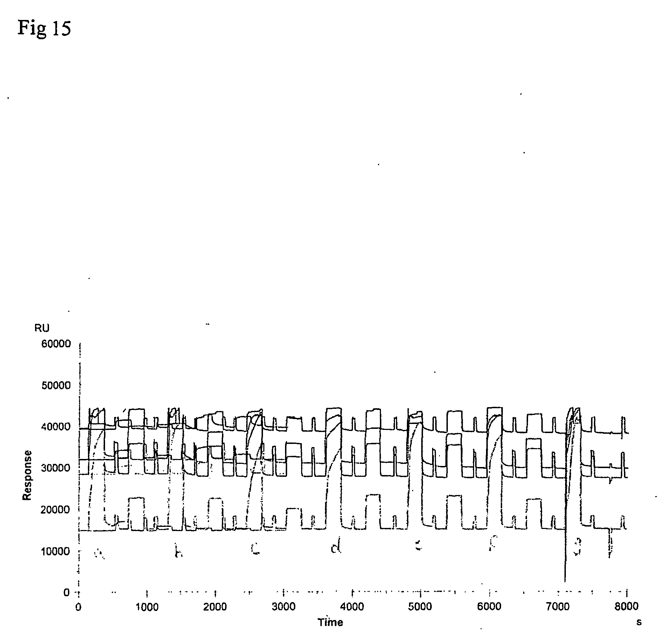

Thus, the present invention relates also to a method for determination of different forms of HGF in ulcer secrete by BIACORE. Ulcer secrete from patients is collected and diluted in buffer such as sodium chloride, distilled water or PBS containing 1-5% protease inhibitor. Carboxymethyl dextran may be added to the preparation (fig 15). In the same manner, concentration and quality of different forms of HGF in the other body fluids might be determined by BIACORE.

In one embodiment, a multichannel device is employed. For example, the channels in the biosensor chip may respectively contain immobilized human anti-HGF monoclonal antibodies, human c-met protooncogen receptor recombinant, and human HGF recombinant. A fourth channel may be used as a control channel. The multichannel device allows one sample to interact with different ligands in the respective channels at the same time. One skilled in the art will appreciate that other ligands may be employed as suitable. The following process may be used for immobilization of ligands: carboxymethylated dextran layer on the sensor chip surface is activated by derivatization with NHS (*N*-hydroxysuccinimide) mediated by EDC (*N*-ethyl-*N'*-(3-diethylaminopropyl) carbodiimide) through injection of 35 µl 0.2M EDC containing 50 mM NHS at an operating temperature of 25 °C giving a sensor response between 10000-30000 response unit (RU). This activation procedure is repeated for all surface flow cells on a single biosensor chip. Monoclonal antibody against HGF (500 µg/ml) is diluted, 1:10 with Acetate pH 4.5 and injected at a flow rate of 5 µl-min⁻¹ in 2-7 minutes. Met proto-oncogene receptor recombinant (100µg/ml) is diluted 1:5 with Acetate pH 4.5 and injected at a flow rate of 5 µl-min⁻¹ in 2-7 minutes. HGF recombinant (5ug/ml) is diluted 1:3 with Acetate pH 4.5 and injected at a flow rate of 5 µl-min⁻¹ in 2-7 minutes. After covalent coupling of the polypeptides, remaining dextran-conjugated NHS-esters are deactivated with 35 µl ethanolamine/hydrochloride at pH 8.5. Flow cells are then washed with five injections with 5µl of a combination of Nacl 1M and Glycin pH 2.0, 1:1.

As some body fluids such as feces and ulcer secrete contain high amounts of protease that might damage the immobilised channels, protease inhibitor (with specific inhibition of serine, cysteine, aspartic proteases and aminopeptidases, containing 4-(2-aminoethyl)benzenesulfonyl fluoride (AEBSF), pepstatinA, E-64, bestatin, leupeptin, and aprotinin but no metal chelators) 1-5% is added to these fluids. A combination of sodium hydroxide 1M and Glycine pH 2, 1:1 at a flow rate of 5 µl-min⁻¹ in one minute was used.

By the present method, different channels of a chip in Biacore apparatus may be immobilized by different ligands to HGF; monoclonal as well as polyclonal antibodies against HGF, c-met protooncogene HGF receptor and dextran may be employed.

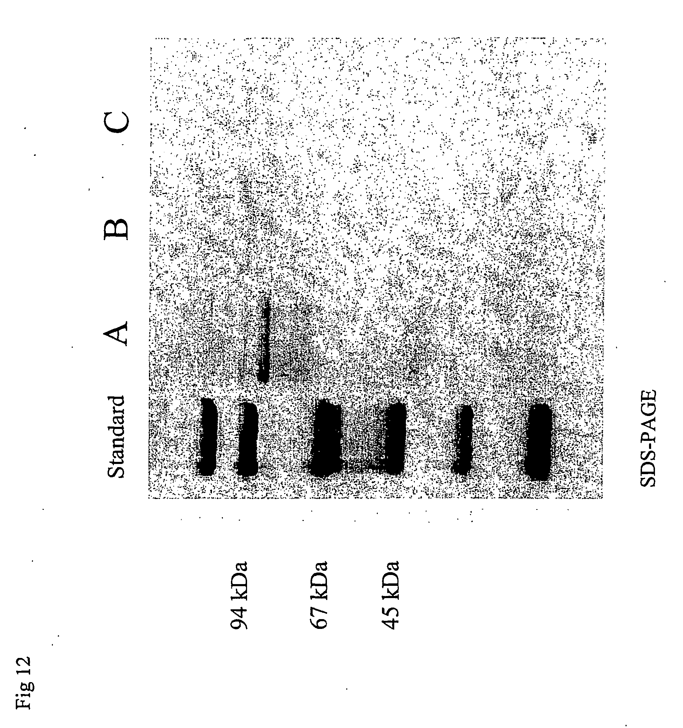

The following description is valid in different body fluids in order to distinguish between different causes of disease:
***In the case of infection*:** In different organs, the levels of HGF are increased locally at the site of infection. The whole amount of protein might be detected by ELISA. Using Biacore technology, detection of the level of interaction (signals) to monoclonal as well as polyclonal antibodies to HGF immobilized to the chip, is high and it correlates positively to the results obtained by ELISA. The increased immobilization level (by adding the concentration of immobilized antibody or the time of immobilization) results in increased signal in these channels. The signals obtained by interaction of HGF to c-met protooncogene receptor immobilized to the channel might be high but increase of immobilization level of c-met does not result in increased signal in c-met channel. The signal obtained from interaction of protein to inactivated dextran channel is always high. Adding carboxymethyl dextran to the samples at least 10 minutes prior to analysis may diminish this signal. The HGF protein may be biologically active and the activity is stopped by adding anti-HGF antibodies to the sample. By immobilization of the channels in a chip by monoclonal antibodies against HGF in different levels, a positive correlation between signals and immobilization level in results is observed. At the same chip, a high signal in the dextran channel is observed.
***In the case of chronic inflammation:*** In spite of high amounts of HGF in samples that might be found by ELISA, non-significant correlation between ELISA and the results obtained by Biacore is observed. It might be no or very low signals detected by Biacore that shows a weak interaction to the ligands. The interaction to c-met protooncogene receptor might be high and the signals correlate positively to the level of immobilization. There is low signal at the dextran channel. Adding carboxymethyl dextran to the samples at least 10 minutes prior to analysis might not diminish the signal at the daxtran channel. The protein might be biologically inactive (Figure 12).

### Example 1

### High hepatocyte growth factor levels in feces during acute infectious gatroenteritis

**Fig 1****.** Shows the individual levels of feces HGF in different groups: Bact = bacterial gastroenteritis, Virus = viral gastroenteritis, NS-D = non-infectious acute diarrhoea, Contr = healthy control group. Seventy patients admitted to the Department of Infectious Diseases because of acute non-treated gastroenteritis were included in a prospective study. Feces from 30 patients with culture verified gastroenteritis caused by Campylobacter jejuni, Clostridium difficile and Salmonella, 10 patients with gastroenteritis caused by rotavirus, 1 patient with Blastocystis hominis, 8 patients with probable infectious gastroenteritis, 10 patients with non-infectious diarrhea and 11 healthy controls were analyzed for detection of HGF by ELISA. There were no significant differences in feces HGF concentrations in the patients with gastroenteritis caused by Clostridium difficile, Campylobacter jejuni or Salmonella. These three are therefore treated as a single group (bacterial infections). Using ANOVA, four statistically significantly different groups could be identified: (1) bacterial infections with high concentrations, (2) viral infections with moderate concentrations, (3) non-infectious acute diarrhea with an average level of HGF slightly above the normal range (patient controls) and (4) normal controls with normal concentrations. There was a complete separation (on an individual basis) between Group 1 on the one hand and Groups 3 (p<0.0001) and 4 on the other with a cut-off value of around 40 ng/g feces, with the viral infections between these groupings (p<0.001).
**Fig 2****.** Is demonstration of HGF (α- and β-chain) in faces of a patient with *Salmonella* enteritis with Western blotting. Proteins from feces were separated by sodium dodecyl sulphate polyacrylamide gel electrophoresis (SDS-PAGE) in 12% acrylamide gels (8 x 5.5 cm) and transferred to a cylindrical polyvinylidene fluoride (PVDF) membrane with electroblotting. The membrane was first incubated with primary goat antibodies against human HGF (1:250, R&D Systems), then with secondary rabbit anti-goat HRP-labelled antibodies (1:40 000, BioRad Laboratories) and finally visualized by enhanced chemiluminescence (Amersham Bioscience) onto an x-ray film.

### Example 2

### The stability of fecal Hepatocyte Growth Factor determination

Fig 3. Relative differences in feces HGF levels compared to baseline (0= separation after 1 hour at room temperature). The included groups are as followed: **Dif 6H RT, 12H RT, 24H RT** : feces kept at room temperature 6, 12, 24 hours after separation. Non-significant differences were observed. **Dif 6H RE, 12H RE, 24H RE and 36H RE**: feces kept in the refrigerator (4-6° C) 6,12, 24 and 36 hours after separation. The differences were not significant. Eight feces samples from patients with diarrhea as well as eight feces samples from normal volunteers were diluted 1:6 in distilled water. The diluted samples were placed in eight tubes (Nunc Cryo Tube, Nunc Brand Products, Denmark). The first tube was immediately placed at -70 °C. Second to fourth tubes were kept for 6, 12 and 24 hours respectively at room temperature prior to freeze storage. The fifth to eighth tubes were kept at 6, 12, 24 and 36 hours respectively at 4 °C (refrigerator) prior to storage. All of the tubes were stored at -70 °C after the period at room temperature or refrigeration period. Storage for 6, 12, or 24 hours at room temperature or refrigeration for 6, 12, 24, or 36 hours (4-6 °C) did not affect feces HGF levels significantly.

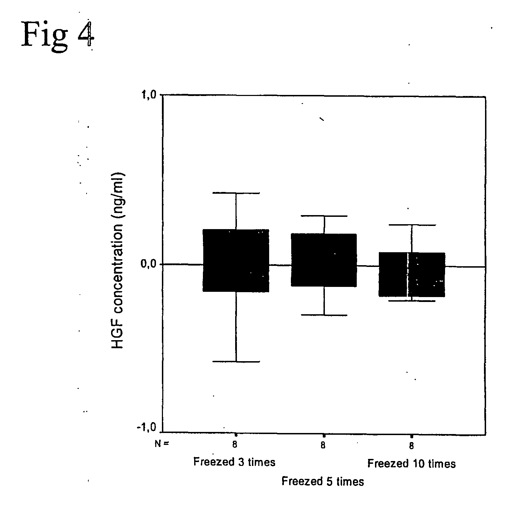

**Fig 4**. Relative differences in feces HGF levels after several (3 times, 5 times and 10 times) freeze-thaw cycles compared to baseline (0= thawed once). The differences were not statistically significant. Eight samples from patients with diarrhea were handled as described above. 250 µl of the diluted suspension in distilled water (see above) was placed in four tubes (Nunc Cryo Tube, Nunc Brand Products, Denmark). The first tube was stored at -70°C immediately. The second tube was freeze-thawed three times (15 minutes at -70°C followed by 30 minutes at room temperature). The third tube was freeze-thawed five times, and the fourth tube freeze-thawed ten times. All of the tubes were stored after the completed freeze-thaw cycles at -70 °C prior to analysis. Feces HGF levels did not change significantly after several freeze-thaw cycles (3,5 and 10 times).

Further we showed that dilution of feces samples in sodium chloride, or phosphate buffer solution (pH 7.4) had no significant effect on feces HGF values. However, dilution in 4% formaldehyde caused a decrease of feces HGF levels at higher concentrations but did not affect the levels at lower concentration. The average difference was not significant. There were no day-to-day variations between feces HGF levels in the healthy volunteers. There were no significant differences between feces HGF concentration in the samples that were prepared immediately after defecation and the samples that stored initially at -20 °C.

There was no significant correlation between feces HGF levels in the patients with infectious gastroenteritis and feces albumin levels in the same sample (Spearman rank correlation, rho 0.17, p=0.50). The albumin level in feces with normal consistence was low (<6 mg/l) in all cases. There was no correlation between presence of blood in the feces (Actim fecal hemoglobin) and feces HGF levels. However the amounts of feces HGF in the patients with positive fecal hemoglobin was slightly but significantly higher than normal control (p<0.05): There was a negative correlation between water content in the feces and total hemoglobin (rho 0.54, p>0.05) in this material. Treatment of feces samples with protease inhibitor did not affect the feces HGF levels.

The levels of feces HGF obtained by ELISA in February 2001 did not differ significantly from the values obtained by ELISA in the same samples in January 2003 (Wilcoxen signed rank test, p=0.90). The levels correlated significantly with each other (Spearman rank correlation, rho 0.90). The calculated intra-assay coefficient of variation was 6.2%; for the inter-assay variation the corresponding value for feces was 16%.

### Example 3

### The role of HGF in restitution of damaged tissue in chronic leg ulcers might be indicated by up-regulation of c-met receptor

**Fig 5****.** Shows the expression of c-met HGF receptor in human skin biopsies from one healthy person (c) compared to a patient with chronic leg ulcer before (a) and after HGF treatment (b). Twelve patients (44-89 years old, 8 women) with chronic leg ulcers were included in the study. The patients had suffered from chronic leg ulcers, which had been stable for at least six months. The etiologies of the ulcers were venous insufficiency or a combined venous and a mild arterial insufficiency determined on clinical judgement, and by physiological measurements, i.e. toe and ankle pressure measurements. The patients with type 1 diabetes mellitus, serious arterial insufficiency, HIV and cancer disease or on medication with warfarin, heparin, low molecular heparin or immunosuppressive therapy, were excluded. Skin biopsies were taken and ulcer secrete was gathered. None of the patients suffered from liver or kidney disease. Skin biopsies were taken also from 10 healthy volunteers (all women 30-55 years old). The patients (six in each group) received double-blinded, local application of the prepared gel for seven days. The ulcers were dressed by occlusive dressings for two days, followed by semiocclusive dressings during the rest of the treatment period. The patients with clinical signs of infection in the ulcers were treated by appropriate antibiotics. No other therapy was allowed during this period. At the end of the period the ulcers were photographed and biopsies were taken. Met proto-oncogene receptor (c-met) was expressed on the capillary endothelial cells and the basal membrane epidermis in the skin biopsies. C-met receptor was up-regulated in the ulcer area in all of the patients with chronic leg ulcer (n=12) compared to the healthy skin in controls (n=10). After one week of treatment the *c-met* receptor was down-regulated in all of the patients that had received active HGF preparation (n=6). C-met receptor was unchanged or up-regulated in the patients who had received placebo (Fisher exact test, p=0.018)
**Fig 6**. Is demonstration of HGF (α- and β-chain) in ulcer secrete of a patient with chronic leg ulcer with Western blotting. Ulcer secrete was collected before treatment by absorption of equal amounts of ulcer secretion during 24 hours by 1 cm² absorbable material (Mepilex, Mölnlycke Health Care AB, Box 13080, SE 40252, Göteborg, Sweden) placed under the dressing and then transferred into a flask (scintillation vial 20 ml, Sarstedt AB, 26151, Landskrona, Sweden) containing 5ml sodium chloride 9%. This was then mixed using a Vortex (Vortex-Genie, Scientific Industries Inc., Bohemia, NY 11716, USA). The filtrated suspension was transferred to tubes (Nunc Cryo Tube, Nunc Brand Products, Denmark) and stored at -70°C prior to analysis. Proteins from ulcer secrete were separated by sodium dodecyl sulphate polyacrylamide gel electrophoresis (SDS-PAGE) in 12% acrylamide gels (8 x 5.5 cm) and transferred to a cylindrical polyvinylidene fluoride (PVDF) membrane with electroblotting. The membrane was first incubated with primary goat antibodies against human HGF (1:250, R&D Systems), then with secondary rabbit anti-goat HRP-labelled antibodies (1:40 000, BioRad Laboratories) and finally visualised by enhanced chemiluminescence (Amersham Bioscience) onto an x-ray film. Three bands corresponding to the precursor, α- and β-chain of HGF were detected by western blot with apparent molecular masses of 75, 55 and 35 kDa respectively.
**Fig 7**: Shows the expression of HGF and expression of met proto-oncogene receptor (c-met) in the same patient. Analyses were performed using a biotin-free immunoperoxidase method
(EnVision^{™} detection system, using TechMate 500+ immunostainer, DakoCytomation). Skin biopsies using a punch biopsy instrument 4 mm (Kai Europé GmbH, Germany) were fixed in 4% formalehyd, embedded in paraffin. Tissue sections were cut 4 micrometer and placed on electrostatically charged slides and heated in an oven at 60°C over night. The tissue were then deparaffinized, rehydrated, and pretreated with heat-induced epitope retrieval in Tris-Edta buffer pH 9.0 in a pressure cooker for 7 minutes at full pressure. Primary antibody at use were anti-HGF (Sigma H1896, clone 24612.111 diluted 1/100) and anti-cMET (Novocastra clone 8F11 diluted 1/50). Mouse anti IgGl + IgM was used as negative control. Diaminobenzidine tetrahydrochloride was used for the chromogen brown reaction product, followed by hematoxylin counterstaining, dehydrating, and mounted with coverslip. Ulcer HGF was determined in 9 patients. There was a negative correlation between HGF concentration in the ulcer (rho=70, p<0.01) and c-met expression in this material.

### Example 4

### Surface plasmon resonance (BIA CORE) detection of fecal hepatocyte growth factors during acute and chronic inflammatory bowel diseases

Fig 8. SPR sensogram showing the signal obtained from analysis of feces of patients with infectious gastroenteritis. Note the differences in signal intensity between the control channel and the channel immobilized by mouse anti-human HGF monoclonal antibody.
**Fig 9****.** Histogram showing the differences between signal responses determined by SPR (channel immobilized by mouse anti-human HGF monoclonal antibodies) in different groups of infectious gastroenteritis (n=9, median 1226 RU), normal healthy controls (n=9, median 199 RU), and patients with colon cancer (n=6, median 70 RU). The response is defined as the difference in response signal after and before the injection of the sample minus the difference observed in the reference channel.

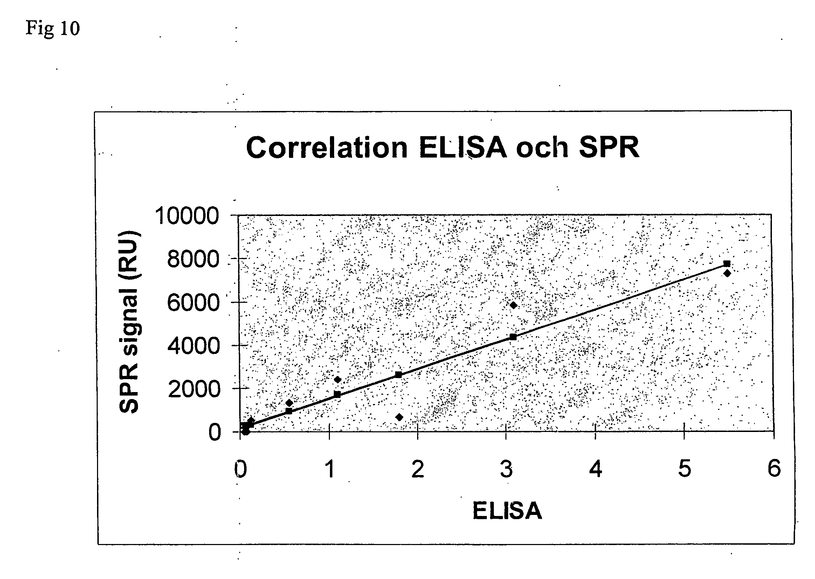

**Fig 10****.** This figure demonstrates the correlation between the concentration of HGF in feces during infectious gasteroenteritis obtained by ELISA and signal responses by SPR (Spearman rank correlation coefficient r=0.93, p<0.01).
**Fig 11****.** Histogram showing the effects of increase of the immobilization levels by increasing the contact time during immobilization (1-3 representing 1, 5, and 10 minutes) (Friedman p<0.01, Wilcoxon signed ranks test between 1 and 5 minutes p=0.058, between 5 and 10 minutes p=0.003, and between 1 and 10 minutes p=0.003) during infectious gasteroenteritis. The monoclonal anti-HGF (500 µg/ml) was diluted 1:10 in 10 mM acetate buffer pH 4.5. The activation time was 7 min, followed by a 1,5 and 10 min ligand injection respectively. Deactivation of remaining active esters was performed by a 7min injection of ethanolamine/hydrochloride at pH 8.5. A flow rate of 5 µl/min was used during all immobilizations.
**Fig 12****.** The picture shows SPS-PAGE of feces. A: before purification; B: after purification in an affinity chromatography column immobilized by mouse anti-human HGF monoclonal antibody; C: after purification in an affinity chromatography column immobilized by recombinant human c-met/fc chimera.
   The affinity chromatography columns (Hi-trap Amersham Biosciences) were immobilized with monoclonal anti-human HGF and recombinant human HGF receptor (c-MET)/Fc chimera (R&D Systems) respectively. Feces samples were reconstituted in distilled water. The samples were applied to the 500 µl centrifugal tubes (Amicon Ultra, Millipore, S.A.S. Molsheim, France) and centrifuged at 4000 G for one hour. SDS-PAGE was performed with the feces samples before and after purification and filtration, using a 4% stacking gel and a 12% running acrylamide gel [12]. For size estimation prestained protein standard (Sigma Aldrich) was electrophoresed simultaneously.

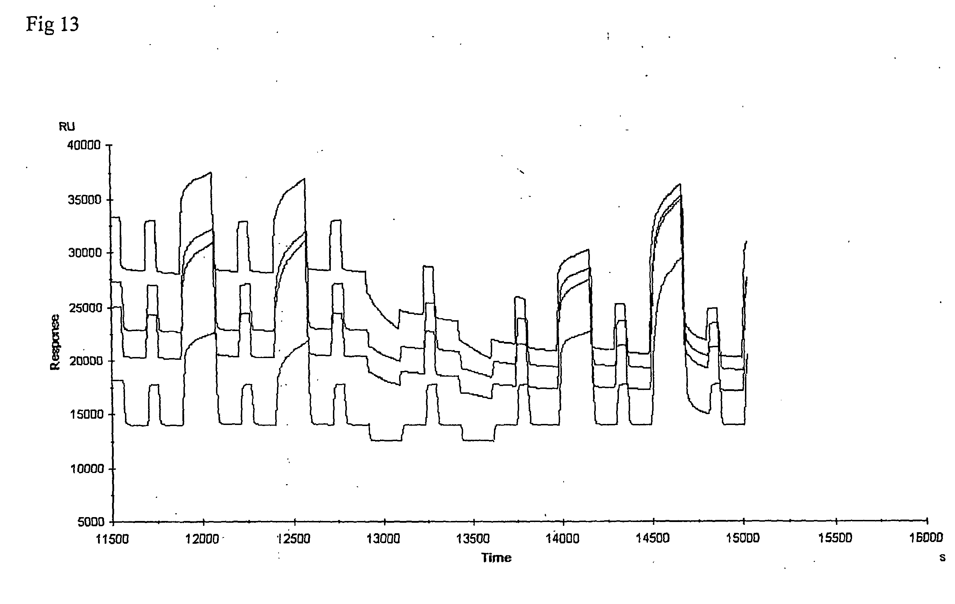

**Fig 13**. Shows an SPR sensogram from feces analysis. The flow cells were immobilized by recombinant Met proto-oncogene receptor (20 µg/ml), monoclonal anti-HGF (50 µg/ml) and HGF recombinant (3 ug/ml) respectively. The last channel (lowest line) was used as a reference to monitor the response due to buffer and unspecific interactions. Every other signal is the regeneration signal (1:1 combination of 1M Nacl and Glycine pH 2.0). The signals at time limits 12000, 12500, 14100 and 14600 seconds belong to the patients with infectious gastroenteritis. The signals at time limits 13000 and 13500 seconds belong to feces of normal healthy control. Please note the decline in the base line of the flow cells at the time limit 13000 when a feces sample of healthy control was injected that did not contain protease inhibitor.
**Fig 14****.** Histogram showing the differences between
   the SPR signal from feces between patients with infectious gasteroenteritis caused by *Clostridium deficile* (n=10) and patients with inflammatory bowel diseases (IBD: crohn and ulcering cholitis, n=33). During infection a more affinity between HGF and monoclonal antibody against HGF is observed compared to the patients with IBD (non-significant). The affinity of HGF found in infectious gastroenteritis i feces has a significant more affinity to dextran channel (p<0.01) compared to IBD. The SPR channels are immobilized as described previously by c-met protooncogene HGF receptor, monoclonal antibody against HGF and monoclonal antibody against TGF beta (R&D, 1mg/mL, diluted 1:10 i acetat pH 4.5), as well as dextran channel.

### Example 5

### Surface plasmon resonance (BIACORE) detection of hepatocyte growth factor in ulcer secrete in the patients with chronic leg ulcers,

**Fig 15**: Shows typical sensogram of human ulcer secrete in nine patients with chronic leg ulcers. Please note the individual differences in response units in ordinary and dextran-bounded samples in each patient (a-g). The first channel (red line) is immobilized by met proto-oncogene receptor recombinant. The second channel (blue line) is immobilized by monoclonal anti-HGF antibody. The third channel is immobilized by human HGF recombinant (purple line). The fourth channel is the dextran channel (green line). Biacore 3000 biosensor system (Biacore AB) operated with the BIACORE 3000 Control Software package system version 3.1.1 was used. According to the specifications of the producer of the instrument, 1000 RU corresponds to 1ng/mm2 material at the surface. A combination of sodium hydroxide 1M and Glycine pH 2, 1:1 at a flow rate of 5 µl-min -1 in one minute was used for regeneration. For more details about collection of samples and methods of detection by BIACORE please see above.

### REFERENCES

1. Arakaki N, Kawakami S, Nakamura O, Ohnishi T, Miyazaki H, Ishii T, Tsubouchi H, Daikuhara Y. Evidence for the presence of an inactive precursor of human hepatocyte growth factor in plasma and sera of patients with liver diseases. Hepatology. 1995 ;22:1728-34.
2. Kono S, Nagaike M, Matsumoto K, Nakamura T. Marked induction of hepatocyte growth factor mRNA in intact kidney and spleen in response to injury of distant organs. Biochem Biophys Res Commun, 1992; 186:991-8.
3. Maeda J, Ueki N, Hada T, Higashino K. Elevated serum hepatocyte growth factor/scatter factor levels in inflammatory lung disease. Am J Respir Crit Care Med. 1995;152: 1587-91.
4. Matsumori A, Furukawa Y, Hashimoto T, Ono K, Shioi T, Okada M, Iwasaki A, Nishio R, Sasayama S. Increased circulating hepatocyte growth factor in the early stage of acute myocardial infarction.Biochem Biophys Res Commun, 1996; 221: 391-5.
5. Matsumoto K, Nakamura T. Hepatocyte growth factor: molecular structure and implications for a central role in liver regeneration. J Gastroenterol Hepatol, 1991a; 6: 509-19.
6. Miyazawa K, Shimomura T, Naka D, Kitamura N. Proteolytic activation of hepatocyte growth factor in response to tissue injury. J Biol Chem, 1994; 269: 8966-70.
7. Naka D, Ishii T, Yoshiyama Y, Miyazawa K, Hara H, Hishida T, Kitamura N. Activation of hepatocyte growth factor by proteolytic conversion of a single chain form to a heterodimer. J Biol Chem, 1992; 267: 20114-9.
8. Nayeri F, Brudin L, Darelid J, Nilsson I, Fryden A, Söderström C, Forsberg P. Hepatocyte growth factor (HGF) may act as an early therapeutic predictor in pneumonia. Scand J Infect Dis, 2002;34 : 500-4.
9. Nayeri F, Nilsson I, Brudin L, Fryden A, Söderström C. High hepatocyte growth factor levels in the acute stage of community-acquired infectious diseases. Scand J Infect Dis, 2002;34:127-30.
10. Nayeri F, Brudin L, Nilsson I, Forsberg P. Sample handling and stability of hepatocyte growth factor in serum during infection. CYTOKINE 2002;19:201-5.
11. Nayeri F, Nilsson I, Hagberg L, Brudin L, Roberg M, Söderström C, Forsberg P. Hepatocyte growth factor (HGF) levels in cerebrospinal fluid: a [WW4]comparison between acute bacterial/non-bacterial meningitis. JID, 2000;181: 2092-4.
12. Nayeri F, Millinger E, Nilsson I, Zetterström O, Brudin L, Forsberg P. Exhaled breath condensate and serum levels of hepatocyte growth factor in pneumonia[WW5]. Respiratory Medicine, 2002; 96:115-9.
13. Nayeri F, Strömberg T, Larsson M, Brudin L, Söderström C, Forsberg P. Hepatocyte growth factor might accelerate healing in chronic leg ulcers: a [WW6]pilot study. J Dermatol Treat, 2002;13:81-6.
14. Noji S, Tashiro K, Koyama E, Nohno T, Ohyama K, Taniguchi S, Nakamura T. Expression of hepatocyte growth factor gene in endothelial and Kupffer's cells of damaged rat livers as revealed by in situ hybridization. Biochem Biophys Res Commun, 1990; 173:42-7.
15. Rosen EM, Joseph A, Jin L, Yao Y, Chau MH, Fuchs A, Gomella L, Hastings H, Goldberg ID, Weiss GH, Urinary and tissue levels of scatter factor in transitional cell carcinoma of bladder. J Urol, 1997;157:72-8.
16. Sakaguchi H, Seki S, Tsubouchi H, Daikuhara Y, Niitani Y, Kobayashi K. Ultrastructural location of human hepatocyte growth factor in human liver. Hepatology. 1994;19:1157-63.
17. Taman M, Liu Y, Tolbert E, Dworkin LD. Increased urinary hepatocyte growth factor excretion in human acute renal failure. Clin Nephrol, 1997; 48:241-5.
18. Ueda T, Takeyama Y, Hori Y, Nishikawa J, Yamamoto M, Saitoh Y. Hepatocyte growth factor in assessment of acute pancreatitis: comparison with C-reactive protein and interleukin-6. J Gastroenterol. 1997;32:63-70.
19. Yamanouchi H, Fujita J, Yoshinouchi T, Hojo S, Kamei T, Yamadori I, Ohtsuki Y, Ueda N, Takahara J. Measurement of hepatocyte growth factor in serum and bronchoalveolar lavage fluid in patients with pulmonary fibrosis. Respir Med. 1998;92:273-8.
20. Yanagita K, Nagaike M, Ishibashi H, Niho Y, Matsumoto K, Nakamura T. Lung may have an endocrine function producing hepatocyte growth factor in response to injury of distal organs. Biochem Biophys res Commun, 1992; 182:802-9.
21. Yanagita K, Matsumoto K, Sekiguchi K, Ishibashi H, Nihol Y, Nakamura T. Hepatocyte growth factor may act as a pulmotrophic factor on lung regeneration after acute ling injury. J Biol Chem, 1993;268:21212-7.

## Claims

1. An *in* vitro method for diagnosing an inflammatory disorder in an individual, comprising contacting a body fluid sample from the individual with an immunoassay device to determine levels of different forms of hepatocyte growth factor (HGF) in the sample, wherein the different forms of HGF are differentiated by their respective affinity to dextran, comprising determining the level of HGF having affinity to a HGF-specific antibody and/or HGF-receptor and the level of HGF having affinity for dextran, wherein the presence of a form of HGF having no or little affinity for dextran is indicative of a chronic inflammation.

2. The method of claim 1, wherein the body fluid sample comprises whole blood, serum, plasma, stool, urine, cerebrospinal fluid, bronchoalveolar lavage, sputum, exhaled breath condensate, semen, saliva, joint fluid or ulcer secrete.

3. The method of claim 1, wherein the method determines the relative levels of single chain HGF precursor and at least one other form of HGF.

4. The method of claim 1, wherein the immunoassay device employs immobilized anti-HGF monoclonal antibody.

5. The method of claim 1, wherein the immunoassay device employs immobilized HGF receptor.

6. The method of claim 5, wherein the HGF receptor comprises met proto oncogene receptor (c-met).

7. The method of claim 1, wherein the immunoassay device employs immobilized carboxymethyl dextran.

8. The method of claim 1, wherein the immunoassay device employs separately immobilized anti-HGF monoclonal antibody, met proto-oncogene receptor (c met), and carboxymethyl dextran.

9. The method of claim 1, wherein the body fluid sample is contacted with dextran prior to contact with the immunoassay device.

10. The method of claim 1, wherein a biosensor produces an electrical signal proportional to each bound analyte.

11. The method of claim 1, wherein the inflammatory disorder comprises bowel disease. CNS disorder, lung disease or injury, kidney disorder, periodontal disorder, peritoneum, pericardium, pleura, or joint disorder.

12. The method of claim 1, wherein the inflammatory disorder comprises acute gastroenteritis.

13. The method of claim 1, wherein the inflammatory disorder comprises inflammatory bowel disease.

14. An immunoassay device for diagnosing an inflammatory disorder in an individual, comprising at least two test areas having immobilized respectively therein reagent for binding different forms of hepatocyte growth factor (HGF), wherein the different forms of HGF are differentiated by their affinity to dextran.

15. The immunoassay device of claim 14, wherein the two tests areas respectively comprise immobilized anti-HGF monoclonal antibody and immobilized HGF receptor.

16. The immunoassay device of claim 15, wherein the HGF receptor comprises met proto-oncogene receptor (c-met).

17. The immunoassay device of claim 14, further comprising an electrical transducer operable to translate a level of bound analyte in a test area into a measurable electrical signal.

18. The immunoassay device of claim 14, comprising a surface plasmon resonance device for translating a level of bound analyte in a test area into a measurable electrical signal.

19. The immunoassay device of claim 14, further comprising a third test area having an additional reagent immobilized therein.

20. The device of claim 19, wherein the additional reagent comprises carboxymethyl dextran.

21. An in vitro method for evaluating an inflammatory disorder in an individual, comprising contacting a body fluid sample from the individual with an immunoassay device to determine levels of different forms of hepatocyte growth factor (HGF) in the sample, wherein the different forms of HGF are differentiated by their respective affinity to dextran, comprising determining the level of HGF having affinity for a HGF-specific antibody and/or HGF-receptor and the level of HGF having affinity for dextran, and wherein the presence of a form of HGF having no or little affinity for dextran is indicative of a chronic inflammation.

22. The method of claim 21, wherein the disorder is skin ulcer.

## Patentansprüche

1. *In vitro*-Verfahren zur Diagnose einer entzündlichen Erkrankung in einem Individuum, umfassend das Inkontaktbringen einer Körperflüssigkeitsprobe von dem Individuum mit einer Immuntestvorrichtung, um die Spiegel verschiedener Formen des Hepatocyten-Wachstumsfaktors (HGF) in der Probe zu bestimmen, wobei die verschiedenen Formen des HGF durch ihre jeweilige Affinität zu Dextran unterschieden werden, umfassend die Bestimmung des Spiegels an HGF, welcher Affinität zu einem HGF-spezifischen Antikörper und/oder HGF-Rezeptor hat, und des Spiegels an HGF, welcher Affinität zu Dextran hat, wobei die Anwesenheit einer Form von HGF, welche keine oder geringe Affinität zu Dextran hat, ein Hinweis auf eine chronische Entzündung ist.

2. Verfahren nach Anspruch 1, wobei die Körperflüssigkeitsprobe Vollblut, Serum, Plasma, Stuhl, Urin, Cerebrospinalflüssigkeit, Bronchoalveolarspülung, Auswurf, ausgeatmetes Atemkondensat, Sperma, Speichel, Gelenkflüssigkeit oder Geschwürsekret umfasst.

3. Verfahren nach Anspruch 1, wobei das Verfahren die relativen Spiegel an einzelkettigem HGF-Vorläufer und mindestens einer anderen Form von HGF bestimmt.

4. Verfahren nach Anspruch 1, wobei die Immuntestvorrichtung immobilisierten Anti-HGF-monoclonalen Antikörper einsetzt.

5. Verfahren nach Anspruch 1, wobei die Immuntestvorrichtung immobilisierten HGF-Rezeptor einsetzt.

6. Verfahren nach Anspruch 5, wobei der HGF-Rezeptor MET-Proto-Onkogen-Rezeptor (c-met) umfasst.

7. Verfahren nach Anspruch 1, wobei die Immuntestvorrichtung immobilisiertes Carboxymethyldextran einsetzt.

8. Verfahren nach Anspruch 1, wobei die Immuntestvorrichtung gesondert immobilisierten Anti-HGF-monoclonalen Antikörper, Met-Proto-Onkogen-Rezeptor (c-met) und Carboxymethyldextran einsetzt.

9. Verfahren nach Anspruch 1, wobei die Körperflüssigkeitsprobe vor dem Kontakt mit der Immuntestvorrichtung mit Dextran in Kontakt gebracht wird.

10. Verfahren nach Anspruch 1, wobei ein Biosensor proportional zu jedem gebundenen Analyten ein elektrisches Signal erzeugt.

11. Verfahren nach Anspruch 1, wobei die entzündliche Erkrankung Darmerkrankung, Erkrankung des ZNS, Lungenerkrankung oder -verletzung, Nierenerkrankung, Parodontalerkrankung oder Erkrankung des Bauchfells, des Perikards, der Pleura oder der Gelenke umfasst.

12. Verfahren nach Anspruch 1, wobei die entzündliche Erkrankung akute Gastroenteritis umfasst.

13. Verfahren nach Anspruch 1, wobei die entzündliche Erkrankung entzündliche Darmerkrankung umfasst.

14. Immuntestvorrichtung zur Diagnose einer entzündlichen Erkrankung in einem Individuum, umfassend mindestens zwei Testbereiche, welche jeweils darin immobilisiertes Reagenz zur Bindung verschiedener Formen vom Hepatocyten-Wachstumsfaktor (HGF) haben, wobei die verschiedenen Formen von HGF durch ihre Affinität zu Dextran unterschieden werden.

15. Immuntestvorrichtung nach Anspruch 14, wobei die zwei Testbereiche immobilisierten Anti-HGF-monoclonalen Antikörper bzw. immobilisierten HGF-Rezeptor umfassen.

16. Immuntestvorrichtung nach Anspruch 15, wobei der HGF-Rezeptor Met-Proto-Onkogen-Rezeptor (c-met) umfasst.

17. Immuntestvorrichtung nach Anspruch 14, ferner umfassend einen elektrischen Wandler, welcher zur Umwandlung eines Spiegels an gebundenem Analyten in einem Testbereich in ein messbares elektrisches Signal eingerichtet ist.

18. Immuntestvorrichtung nach Anspruch 14, umfassend eine Oberflächenplasmonresonanzvorrichtung zur Umwandlung eines Spiegels an gebundenem Analyten in einem Testbereich in ein messbares elektrisches Signal.

19. Immuntestvorrichtung nach Anspruch 14, ferner umfassend einen dritten Testbereich, welcher darin ein zusätzliches immobilisiertes Reagenz hat.

20. Vorrichtung nach Anspruch 19, wobei das zusätzliche Reagenz Carboxymethyldextran umfasst.

21. In-vitro-Verfahren zur Bewertung einer entzündlichen Erkrankung in einem Individuum, umfassend das Inkontaktbringen einer Körperflüssigkeitsprobe von dem Individuum mit einer Immuntestvorrichtung, um die Spiegel verschiedener Formen des Hepatocyten-Wachstumsfaktors (HGF) in der Probe zu bestimmen, wobei die verschiedenen Formen des HGF durch ihre jeweilige Affinität zu Dextran unterschieden werden, umfassend das Bestimmen des Spiegels an HGF, welcher Affinität zu einem HGF-spezifischen Antikörper und/oder HGF-Rezeptor hat, und des Spiegels an HGF, welcher Affinität zu Dextran hat, wobei die Anwesenheit einer Form von HGF, welche keine oder geringe Affinität zu Dextran hat, ein Hinweis auf eine chronische Entzündung ist.

22. Verfahren nach Anspruch 21, wobei die Erkrankung ein Hautgeschwür ist.

## Revendications

1. Procédé in vitro de diagnostic d'un trouble inflammatoire chez un individu, comprenant la mise en contact d'un échantillon de fluide corporel de l'individu avec un dispositif d'immunoessai pour déterminer les taux de différentes formes du facteur de croissance des hépatocytes (HGF) dans l'échantillon, dans lequel les différentes formes de l'HGF sont différentiées par leur affinité respective vis-à-vis du dextrane, comprenant la détermination du taux de l'HGF ayant une affinité vis-à-vis d'un anticorps spécifique de l'HGF et/ou d'un récepteur de l'HGF et du taux de l'HGF ayant une affinité pour le dextrane, dans lequel la présence d'une forme de l'HGF ayant pas ou peu d'affinité pour le dextrane est indicative d'une inflammation chronique.

2. Procédé selon la revendication 1, dans lequel l'échantillon de fluide corporel comprend du sang entier, du sérum, du plasma, des matières fécales, de l'urine, du liquide céphalo-rachidien, un lavage broncho-alvéolaire, un crachat, un condensat d'haleine expirée, du sperme, de la salive, un fluide articulaire ou une sécrétion ulcéreuse.

3. Procédé selon la revendication 1, le procédé déterminant les taux relatifs du précurseur de l'HGF à chaîne unique et d'au moins une autre forme de l'HGF.

4. Procédé selon la revendication 1, dans lequel le dispositif d'immunoessai emploie un anticorps monoclonal anti-HGF immobilisé.

5. Procédé selon la revendication 1, dans lequel le dispositif d'immunoessai emploie un récepteur de l'HGF immobilisé.

6. Procédé selon la revendication 5, dans lequel le récepteur de l'HGF comprend un récepteur du proto-oncogène met (c-met).

7. Procédé selon la revendication 1, dans lequel le dispositif d'immunoessai emploie du dextrane carboxyméthylé immobilisé.

8. Procédé selon la revendication 1, dans lequel le dispositif d'immunoessai emploie séparément un anticorps monoclonal anti-HGF immobilisé, un récepteur du proto-oncogène met (c met) et du dextrane carboxyméthylé.

9. Procédé selon la revendication 1, dans lequel l'échantillon de fluide corporel est mis en contact avec du dextrane avant le contact avec le dispositif d'immunoessai.

10. Procédé selon la revendication 1, dans lequel un biocapteur produit un signal électrique proportionnel à chaque analyte lié.

11. Procédé selon la revendication 1, dans lequel le trouble inflammatoire comprend une maladie intestinale, un trouble du SNC, une maladie ou lésion pulmonaire, un trouble rénal, un trouble périodontique, un trouble du péritoine, du péricarde, de la plèvre ou articulaire.

12. Procédé selon la revendication 1, dans lequel le trouble inflammatoire comprend une gastroentérite aiguë.

13. Procédé selon la revendication 1, dans lequel le trouble inflammatoire comprend une maladie intestinale inflammatoire.

14. Dispositif d'immunoessai pour diagnostiquer un trouble inflammatoire chez un individu, comprenant au moins deux zones d'essai dans lesquelles est respectivement immobilisé un réactif pour lier différentes formes du facteur de croissance des hépatocytes (HGF), dans lequel les différentes formes de l'HGF sont différentiées par leur affinité vis-à-vis du dextrane.

15. Dispositif d'immunoessai selon la revendication 14, dans lequel les deux zones d'essai comprennent respectivement un anticorps monoclonal anti-HGF immobilisé et un récepteur de l'HGF immobilisé.

16. Dispositif d'immunoessai selon la revendication 15, dans lequel le récepteur de l'HGF comprend le récepteur du proto-oncogène met (c-met).

17. Dispositif d'immunoessai selon la revendication 14, comprenant en outre un transducteur électrique apte à traduire un taux d'analyte lié dans une zone d'essai en un signal électrique mesurable.

18. Dispositif d'immunoessai selon la revendication 14, comprenant un dispositif de résonance des plasmons de surface pour traduire un taux d'analyte lié dans une zone d'essai en un signal électrique mesurable.

19. Dispositif d'immunoessai selon la revendication 14, comprenant en outre une troisième zone d'essai dans laquelle est immobilisé un réactif supplémentaire.

20. Dispositif selon la revendication 19, dans lequel le réactif supplémentaire comprend du dextrane carboxyméthylé.

21. Procédé *in vitro* pour évaluer un trouble inflammatoire chez un individu, comprenant la mise en contact d'un échantillon de fluide corporel de l'individu avec un dispositif d'immunoessai pour déterminer les taux de différentes formes du facteur de croissance des hépatocytes (HGF) dans l'échantillon, dans lequel les différentes formes de l'HGF sont différentiées par leur affinité respective vis-à-vis du dextrane, comprenant la détermination du taux de l'HGF ayant une affinité pour un anticorps spécifique de l'HGF et /ou un récepteur de l'HGF et du taux de l'HGF ayant une affinité pour le dextrane, et dans lequel la présence d'une forme de l'HGF ayant pas ou peu d'affinité pour le dextrane est indicative d'une inflammation chronique.

22. Procédé selon la revendication 21, dans lequel le trouble est un ulcère de la peau.
